# EUROPEAN PATENT SPECIFICATION

(11) **EP 1 009 308 B1**
(45) Date of publication and mention of the grant of the patent: **27.04.2005**
(21) Application number: 98908671.5
(22) Date of filing: 23.02.1998
(51) Int. Cl.: A61B 17/60

(54) **JOINT SUPPORT**
GELENKSTÜTZE
SOUTIEN D'ARTICULATION

(30) Priority: 22.02.1997 US 803805
(43) Date of publication of application: 21.06.2000
(73) Proprietor: Slocum, D. Barclay, Eugene, Oregon 97408 (US)
(72) Inventor: Slocum, D. Barclay, Eugene, Oregon 97408 (US)
(74) Representative: Zeitler & Kollegen
(86) International application number: PCT/US1998/003509
(87) International publication number: WO 1998/036698

(56) References cited:
- US-A- 4 338 927
- US-A- 4 604 996
- US-A- 4 604 997
- US-A- 4 696 293
- US-A- 4 922 896

## Description

### Technical Field

The invention relates generally to devices for supporting a joint in the limb of an animal, namely the joint between the tibia and the tarsus. The invention is attachable to desired bones of the animal via bone pins that penetrate the bone and extend through the animal flesh. Put another way, the joint support of the present invention is an external bone-joint fixator. The joint support has certain adjustable features, but once it is placed in an adjusted position, the joint support has a single degree of freedom about a hinge. When attached to pre-selected bones of an animal so that the hinge overlies a joint located between the bones, the joint support limits movement of that joint to the single degree of freedom of the support.

### Background Art

It is known to attach an external, mechanically hinged joint brace to a human limb, with the hinge overlying a joint in that limb. For example, U.S. Patent No. 5 376 091 discloses several such braces. Another such conventional brace is known as the Richards-Illizarov System.

US-4 696 293 discloses a hinged external fixator which holds bony parts such as the tarsus by means of screws or pins embedded in the bony parts. Elongated structural members are pivotally connected, wherein both members are connected to a hinge in a radial manner. This hinged external fixator is designed for arms or legs of a human body.

From US 4 604 997 is known an orthopedic mini external fixation device comprising two articulated members having two clamps, each of which is carried by a different one of the articulated members. The preamble of claim 1 is based on the features of this fixation device.

However, conventional joint braces have been constructed only for application to human joints. Such joint braces are ineffective for application to non-human animals, at least in part because they are too complicated, and thus likely to be damaged or otherwise disturbed by the animal while wearing the brace. Conventional joint braces are also structurally inadequate for joints in non-human animal limbs.

### Summary of the Invention

The present invention provides a joint support that effectively supports the joints of animals, and is particularly suited for use with domesticated animals such as dogs and cats. The invention generally includes a hinge, a radial support element attached to one side of the hinge, and a transverse support element attached to the other side of the hinge. The transverse element rotates relative to the radial element, with the transverse element rotating tangentially about the hinge. Tangential rotation means that the axial portion of the transverse element closest to the axis of rotation of the hinge defines substantially a circle as it rotates around the hinge, and the longitudinal axis of the transverse element is about tangent to the defined circle.

The hinge includes what is referred to as a radial block and a transverse block, with the radial block being that portion of the hinge that is attached to the radial support element, and the transverse block being that portion of the hinge that is attached to the transverse support element. The radial support element is fixed to the radial block, and the transverse support element extends through the transverse block to define an anterior portion and a posterior portion of the transverse element. In one embodiment of the present invention, the transverse element is releasably retained within the transverse block by a setscrew so that the proportion of the length of the anterior portion to the length of the posterior portion can be adjusted as necessary. Alternatively, the transverse element can be permanently affixed to the transverse block. In either embodiment, both the transverse and radial elements can be cut to a ny desired length.

For supporting the tarsus and metatarsus of an animal relative to the tibia the joint support according to the invention comprises: a pintle having a longitudinal axis; a radial block as one of the hinge blocks having formed therein a pintle bore, the pintle bore including a longitudinal axis, wherein the radial block is mounted on the pintle; and a transverse block as one of the hinge blocks having formed therein a pintle bore, the pintle bore including a longitudinal axis, wherein the transverse block is mounted on the pintle; wherein the radial element being operatively connected to the radial block so that the longitudinal axis of the radial element is substantially perpendicular to the longitudinal axis of the pintle, and so that the longitudinal axis of the radial element substantially intersects the longitudinal axis of the pintle; wherein the transverse element being operatively connected to and extending through the transverse block to define an anterior portion and a posterior portion of the transverse element, with the longitudinal axis of the transverse element being substantially offset from the longitudinal axis of the pintle and substantially coplanar with the longitudinal axis of the radial element; wherein, in use, the radial element is affixed to the tibia and the transverse element is affixed to the tarsus and metatarsus, with the posterior portion of the transverse element being generally affixed to the tarsus and the anterior portion of the transverse element being generally affixed to the metatarsus.

### Brief Description of the Drawings

Fig. 1 is a top view of the present invention, showing the support elements and hinge, but exclusive of attached bone pins and clamps;
Fig. 2 is a side elevation of the joint support shown in Fig. I, illustrating in schematic form the attached bone pins;
Fig. 3 is a bottom view of the joint support shown in Fig. 2;
Fig. 4 is a cross-sectional view of the joint support shown in Fig. 2, taken generally along line 4-4 in Fig. 2, and omitting attached bone pins;
Fig. 5 is a cross-sectional view of the joint support shown in Fig. 4, taken generally along line 5-5 in Fig. 4;
Fig. 6 is a cross-sectional of an alternative embodiment of the joint support shown in Fig. 4, taken generally along line 6-6 in Fig. 4, showing a alternate method of attaching the support elements to the hinge blocks, and shown with sections of the transverse support element removed; and
Fig. 7 is a medial view of a section of a dog skeleton, showing the tibia, tarsus and metatarsus attached to the joint support of the present invention.

### Detailed Description of the Drawings and Best Mode for Carrying Out the Invention

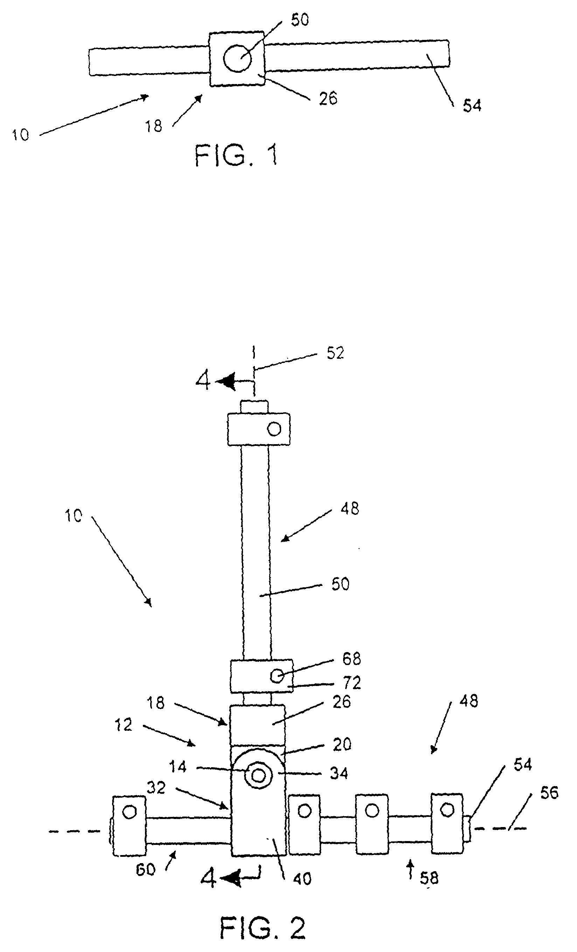

Referring generally to the drawings and focusing particularly on Fig. 2, a joint support according to the present invention is indicated generally at 10. Joint support 10 includes a hinge 12 defined by a pintle 14, preferably with a tubular passage being formed in pintle 14 to define a longitudinal axis or hinge axis 16. Hinge 12 can be further defined to include a pair of hinge bodies.

One such hinge body is a radial block 18, having a tongue 20 in which is formed a pintle bore defining a longitudinal axis. Radial block 18 further includes a body 26 in which is formed a radial bore defining a longitudinal axis. The longitudinal axis from radial bore in body 28 is preferably substantially perpendicular to and intersecting the longitudinal axis of the pintle bore in tongue 20.

Another such block is a transverse block 32, having a tongue 34 in which is formed a pintle bore defining a longitudinal axis. Transverse block 32 further includes a body 40 in which is formed a transverse bore defining a longitudinal axis. The longitudinal axis of transverse bore in body 40 is preferably substantially perpendicular to and offset from the longitudinal axis of pintle bore in tongue 34. At setscrew bore extends through transverse block 32 to open into transverse bore in body 40 and is preferably substantially perpendicular to the transverse bore in body 40.

Joint support 10 includes support structure 48 operatively connected to hinge 12. One element of support structure 48 is a radial element 50 defining a longitudinal axis 52. Radial element 50 is connected to hinge 12 so that radial axis 52 is substantially perpendicular to and intersecting hinge axis 16. Another element of support structure 48 is a transverse element 54, defining a longitudinal axis 56, and in combination with radial element 50, defining a plane of support. Transverse element 54 includes an anterior portion 58 and a posterior portion 60, with anterior portion 58 being defined relative to posterior portion 60 by a line that extends perpendicularly from radial element 50 and intersects 24 longitudinal axis of pintle bore in tongue 20. Longitudinal axis 56 is substantially perpendicular to and offset from hinge axis 16.

In the preferred embodiment, radial element 50 extends into radial bore in body 26, and transverse element 54 extends through transverse bore in body 40. Radial element 50 is fixed to hinge 12, preferably by threading radial element 50 into radial bore in body 26. The threaded engagement provides a relatively secure attachment, particularly when joint 10 is attached to an animal, yet also allows easy substitution of one length of radial element 50 for another.

Other methods of attaching radial element 50 to hinge 12 could be used, including pressing, gluing, brazing or welding radial element 50 into hinge 12. Alternatively, radial element 50 could be fixed relative to hinge 12 by a setscrew, not shown, or element 50 could be formed with an integral pintle bore, also not shown. Transverse element 54 is fixed relative to hinge 12 by a setscrew 62, having a tool socket 64 and defining a longitudinal axis. As with radial element 50, transverse element 54 could be threaded into transverse block 32, or formed with an integral pintle bore, not shown. Other means of pivotally interconnecting radial element 50 to transverse element 54, while preserving the relationship of elements 50 and 54 to the defined axis of pivoting are intended to be within the scope of the claims, set forth below.

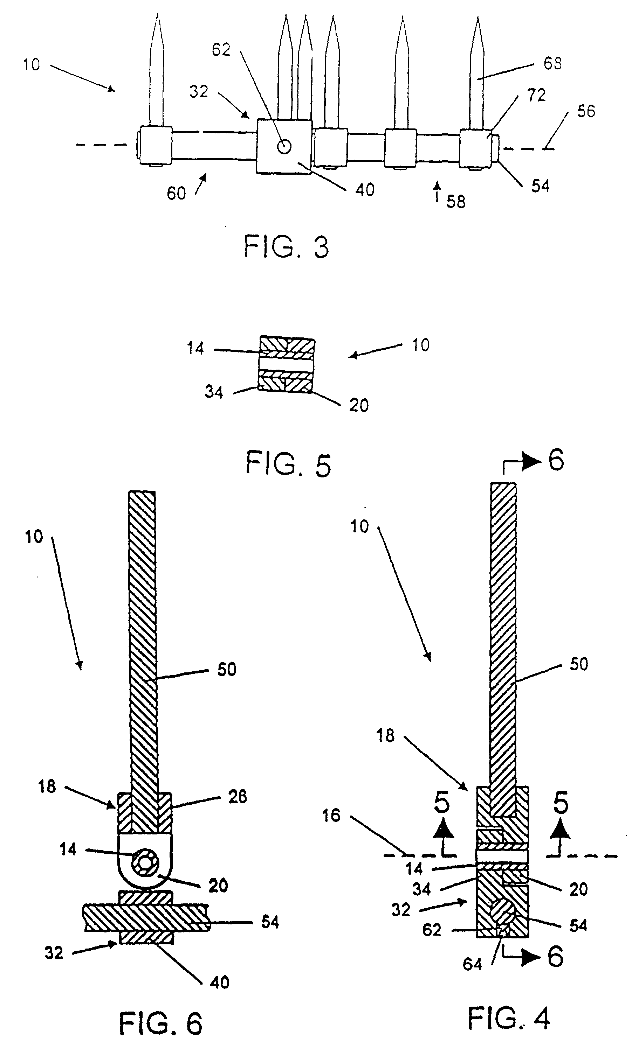

For reference, bone pins are indicated generally at 68, each defining a longitudinal axis that is usually substantially normal to the support plane defined by support structure 48. Joint support 10 can be used with other configurations of bone pins 68. Bone pins 68 are attached to support structure 48 as desired, with clamps 72 as shown in Figs. 2-3.

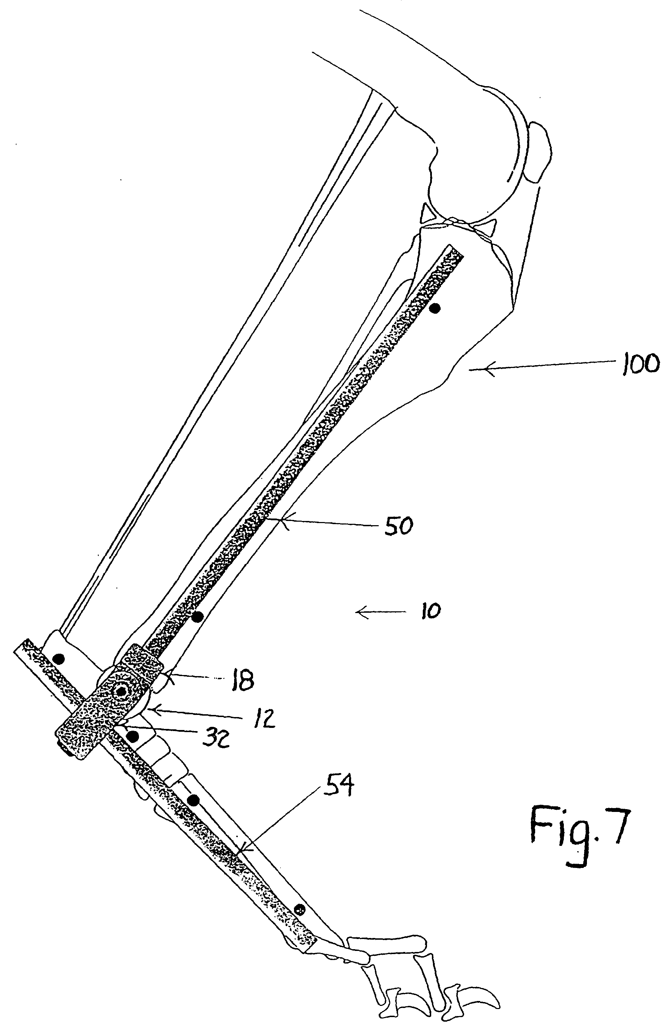

Fig. 7 shows a representative example of joint support 10 being used to support the hock joint of a dog. Pertinent sections of a dog anatomy are shown generally at 100, including a tibia, a tarsus and a metatarsus.

### Industrial Applicability

The joint support of the present invention is applicable in any situation where it is desired to support a movable joint of an animal. It is particularly applicable to the support of the tibia-tarsal joint (hock joint) and the humerus-ulna/radius joint (elbow joint) of a dog or a cat.

## Claims

1. A joint support (10) for attachment to a limb of an animal, the joint support (10) limiting the movement of a joint in that limb to be substantially around a single axis, the joint support (10) comprising:
a pintle (14) having a longitudinal axis which defines a hinge axis (16);
two hinge blocks (18,32) hereinafter referred to as the radial block (18) and the transverse block (32), each having formed therein a pintle bore, which are mounted on and pivot around said pintle (14);
a radial element (50) having a longitudinal axis (52), the radial element (50) being attached to the radial block (18), so that the longitudinal axis (52) of the radial element (50) is substantially perpendicular to the hinge axis (16), and so that the longitudinal axis of the radial element (50) substantially intersects with the hinge axis (16), wherein the radial element (50) is restricted to rotation around the hinge axis (16); and
a transverse element (54) having a longitudinal axis (56), the transverse element (54) being connected to the transverse block (32), so that the longitudinal axis (56) of the transverse element (54) is substantially offset from the hinge axis(16), and is substantially coplanar with the longitudinal axis (52) of the radial element (50);
**characterised in that**,
the transverse element (54) extends through the transverse block (32) to define an anterior portion (58) and a posterior portion (60) of the transverse element (54),
wherein, in use, the radial element (50) is affixed to the tibia and the transverse element (54) is affixed to the tarsus and metatarsus, with the posterior portion (60) of the transverse element (54) being affixed to the tarsus and the anterior portion (58) of the transverse element (54) being affixed to the metatarsus.

2. The joint support (10) according to claim 1, wherein the longitudinal axis (56) of the transverse element (54) is substantially perpendicular to the axis of rotation (16) so that the transverse element (54) is restricted to about tangential rotation around the hinge axis (16).

## Patentansprüche

1. Gelenkstütze (10) zur Befestigung an einem Glied eines Tieres, wobei die Gelenkstütze (10) die Bewegung eines Gelenkes in diesem Glied im wesentlichen um eine einzige Achse einschränkt und folgendes umfasst:
einen Gelenkbolzen (14) mit einer Längsachse, die eine Scharnierachse (16) definiert;
zwei Scharnierblöcke (18, 32), die nachfolgend als Radialblock (18) und Querblock (32) bezeichnet werden, jeweils eine Gelenkbolzenbohrung aufweisen und an den Gelenkbolzen (14) montiert sowie um diesen herum schwenkbar sind;
ein Radialelement (50) mit einer Längsachse (52), das an dem Radialblock (18) befestigt ist, so dass die Längsachse (52) des Radialelementes (50) im wesentlichen senkrecht auf der Scharnierachse (16) steht und im wesentlichen die Scharnierachse (16) schneidet, wobei das Radialelement (50) eingeschränkt ist auf eine Drehung um die Scharnierachse (16); und
ein Querelement (54) mit einer Längsachse (56), das an den Querblock (32) derart angeschlossen ist, dass die Längsachse (56) des Querelementes (54) im wesentlichen versetzt ist zur Scharnierachse (16) und im wesentlichen coplanar ist zur Längsachse (52) des Radialelementes (50);
**dadurch gekennzeichnet, dass**
sich das Querelement (54) durch den Querblock (32) erstreckt und einen vorderen Teil (58) sowie einen hinteren Teil (60) des Querelementes (54) definiert, wobei beim Einsatz das Radialelement (50) an der Tibia befestigt ist und das Querelement (54) an dem Fußglied und dem Mittelfuß befestigt ist, während der hintere Teil (60) des Querelementes (54) an dem Fußglied befestigt ist und der vordere Teil (58) des Querelementes (54) an dem Mittelfuß befestigt ist.

2. Gelenkstütze (10) gemäß Anspruch 1, wobei die Längsachse (56) des Querelementes (54) im wesentlichen senkrecht steht zur Drehachse (16), so dass das Querelement (54) beschränkt ist auf eine tangentiale Drehung um die Scharnierachse (16).

## Revendications

1. Support d'articulation (10) destiné à être attaché à un membre d'un animal, le support d'articulation (10) limitant le mouvement d'une articulation de ce membre à se produire sensiblement autour d'un axe unique, le support d'articulation (10) comprenant :
un pivot (14) ayant un axe longitudinal qui définit un axe de charnière (16) ;
deux blocs de charnière (18, 32), désignés ci-après comme étant le bloc radial (18) et le bloc transversal (32), chacun comportant un perçage de pivot formé en lui-même, et qui sont montés sur ledit pivot (14) en pivotement autour de celui-ci ;
un élément radial (50) présentant un axe longitudinal (52), l'élément radial (50) étant attaché au bloc radial (18) de telle façon que l'axe longitudinal (52) de l'élément radial (50) est sensiblement perpendiculaire à l'axe de charnière (16), et de telle façon que l'axe longitudinal de l'élément radial (50) recoupe sensiblement l'axe de charnière (16), l'élément radial (50) étant restreint à une rotation autour de l'axe de charnière (16) ; et
un élément transversal (54) ayant un axe longitudinal (56), l'élément transversal (54) étant connecté au bloc transversal (32) de telle façon que l'axe longitudinal (56) de l'élément transversal (54) est sensiblement décalé depuis l'axe de charnière (16), et est sensiblement coplanaire avec l'axe longitudinal (52) de l'élément radial (50) ;
**caractérisé en ce que** :
l'élément transversal (54) s'étend à travers le bloc transversal (32) pour définir une portion antérieure (58) et une portion postérieure (60) de l'élément transversal (54), de sorte que, en utilisation, l'élément radial (50) est fixé au tibia et l'élément transversal (54) est fixé au tarse et au métatarse, la portion postérieure (60) de l'élément transversal (54) étant fixée au tarse et la portion antérieure (58) de l'élément transversal (54) étant fixée au métatarse.

2. Support d'articulation (10) selon la revendication 1, dans lequel l'axe longitudinal (56) de l'élément transversal (54) est sensiblement perpendiculaire à l'axe de rotation (16), de sorte que l'élément transversal (54) est restreint à une rotation environ tangentielle autour de l'axe de charnière (16).
